# EUROPEAN PATENT SPECIFICATION

(11) **EP 2 911 635 B1**
(45) Date of publication and mention of the grant of the patent: **06.12.2017**
(21) Application number: 13848333.4
(22) Date of filing: 01.10.2013
(51) Int. Cl.: A61F 13/60, A61F 13/56, A61F 13/505, A61F 13/15

(54) **METHOD AND APPARATUS FOR FORMING AN ADJUSTABLE PANT-LIKE DISPOSABLE UNDERGARMENT WITH LASER CUTTING**
VERFAHREN UND VORRICHTUNG FÜR EINE VERSTELLBARE HOSENÄHNLICHE WEGWERFUNTERWÄSCHE DURCH LASERSCHNEIDEN
PROCÉDÉ ET APPAREIL POUR LA FORMATION D'UN SOUS-VÊTEMENT JETABLE DE TYPE CULOTTE AJUSTABLE AVEC DÉCOUPE LASER

(30) Priority: 24.10.2012 US 201213658868
(43) Date of publication of application: 02.09.2015
(73) Proprietor: Kimberly-Clark Worldwide, Inc., Neenah, Wisconsin 54956 (US)
(72) Inventor: VERBOOMEN, Jason, Andrew, Appleton, Wisconsin 54913 (US); SCHOON, Bradley, William, Oshkosh, Wisconsin 54904 (US); SORENSON, Jesse, Paul, Neenah, Wisconsin 54956 (US); MILBRODT, Paul, George, Neenah, Wisconsin 54956 (US); RHODES, Brian, Keith, Winneconne, WI 54947 (US)
(74) Representative: Davies, Christopher Robert
(86) International application number: PCT/IB2013/059049
(87) International publication number: WO 2014/064562

(56) References cited:
- EP-A1- 1 736 272
- US-A- 5 468 320
- US-A1- 2002 148 557
- US-A1- 2003 029 575
- US-A1- 2004 182 502
- US-A1- 2010 236 703
- US-A1- 2012 143 166
- US-B1- 6 443 938
- US-B1- 6 454 752

## Description

### BACKGROUND

Pant-like disposable undergarments for absorbing human discharges can appear similar in size and shape to regular cloth underwear which is designed to be laundered and reused. A disposable absorbent undergarment is intended to be worn by persons, including infants, toddlers, or adults, and is designed for a single or temporary use and is meant to be disposed of after being used once instead of being laundered or dry cleaned for re-use. Some examples of disposable undergarments include infant diapers, training pants, adult incontinence garments, feminine pants, etc.

Some pant-like disposable absorbent undergarments manufactured today resemble regular cloth underwear in that they have a waist opening and a pair of leg openings. Such pant-like disposable absorbent undergarments can be pulled up around the torso of a wearer in a similar fashion as regular cloth underwear. Still other pant-like disposable absorbent undergarments have an open or flat configuration and are designed to be placed adjacent to a wearer's torso and then rely upon one or more attachment tabs or fasteners to secure the undergarment around the wearer's torso. This design is beneficial for bed bound users who may be immobile or for children who need assistance in securing the undergarment in place. Still other adjustable, pant-like absorbent undergarments contain attachment means for opening and closing the waist opening after the undergarment has been positioned around the wearer's torso. This type of adjustable undergarment has an advantage in that the wearer does not have to remove outer clothing in order to check the status of the undergarment or to remove the undergarment from their body.

One example of such an adjustable, pant-like disposable absorbent undergarment includes a pair of lines of weakness that a user must break to enable adjusting the fit of the undergarment. The lines of weakness usually extend from the waist opening to one of the leg openings and are designed to be broken either prior to positioning the undergarment around the user's torso or while the undergarment is already positioned around the wearer's torso. A pair of fastener assemblies is then utilized to refasten the undergarment so that it is snug about the wearer's torso.

It has been found that a major portion of each of the lines of weakness is visually hidden and some users cannot see them and thereby do not know that they are present. In addition, each line of weakness may be ergonomically hard to tear open by older adults, some of who may be suffering from arthritis. In addition, options that require tearing often indicate to users that the product is damaged or of poor quality when torn.

An adjustable, pant-like disposable absorbent undergarment that includes a fully severed front panel and more readily apparent and more easily accessible fastener mechanisms is needed. Typically, however, the types of consumer goods mentioned above are manufactured on a continuous basis on large scale manufacturing lines. Usually, various raw products or components are formed on, or integrated into, a continuous stream of material, which often includes a web of material that moves in a machine direction through and along the line. As such, it is important to maintain the integrity of the stream of material or web during the process so as to avoid costly downtime. In general, the web is pushed or pulled along the line, so as to put the web in tension. Accordingly, the formation of a fully severed panel, especially along a cross-direction, can increase the risk of breakage. Therefore, it is desirable to maintain the tensile strength of the stream of materials or web as it passes through the process.

Therefore, there is need to provide a method and apparatus for manufacturing pant-like disposable absorbent undergarment that includes a fully or partially severed front panel without causing a problem with the integrity of the stream of material or web during the process.

Related art includes US 2012/0143166 which discloses a fastening tab and method of making the same. US 2002/148557 discloses a method of assembling personal care absorbent articles. US 2004/182502 discloses a method of forming a disposable refastenable absorbent article. EP 1736272 discloses a method and device for laser treating articles, in particular sanitary products.

### SUMMARY

Generally, a method and apparatus for producing a pre-fastened adjustable pant-like disposable absorbent undergarment including a fully or partially severed front body panel is disclosed. The method includes the steps of providing a moving web and passing it through a fastener attachment station to define a leading portion of the moving web and a trailing portion of the moving web at a location under the fastener assembly. Finally, the moving web is then passed through a laser cutting station to cut the web, such that the leading portion and trailing portion remains bridged together by the fastener assembly. Desirably, the fastener assembly is not damaged when the moving web is cut with the laser. However, in other embodiments, the fastener assembly is weakened by the laser as the web is cut. A weaker fastener assembly provides for a more flexible fastener assembly that could be easier for a consumer to manipulate.

In one embodiment, cutting the moving web involves completely separating the leading portion of the moving web and the trailing portion of the moving web. This may be done with a single cut across the web substantially in the cross-machine direction.

In another embodiment, cutting the moving web involves creating a line of weakness between the leading portion of the moving web and the trailing portion of the moving web. This could be a series of intermittent perforations or scoring the web to provide a weakened area.

These features will be described in greater detail herein. Further, it is to be understood that both the foregoing general description and the following detailed description are exemplary and are intended to provide further explanation of the invention claimed.

### BRIEF DESCRIPTION

Figure 1 depicts a method of producing a pre-fastened adjustable pant-like disposable absorbent undergarment including a fully severed front body panel.
Figure 2 depicts an apparatus for producing a pre-fastened adjustable pant-like disposable absorbent undergarment including a fully severed front body panel.
Figure 3 depicts a front perspective view of one example of a garment, the garment shown in a pre-fastened, pant-like configuration.
Figure 4 depicts a front perspective view of one example of a garment, the garment shown in an unfastened, pant-like configuration.
Figure 5 depicts a plan view of the garment of Figure 1, the garment shown in an unfastened, laid-open, relaxed configuration.

### DETAILED DESCRIPTION

Reference to the Figures shall be made in describing various embodiments. It should be noted that the embodiments depicted in the Figures and described herein are merely representative examples. The various embodiments are suitable for use in conjunction with disposable absorbent undergarments such as refastenable adult incontinence underwear, pre-fastened disposable diapers, refastenable disposable training pants or swim pants, refastenable disposable enuresis garments, and the like. For illustration purposes, various embodiments shall be described in conjunction with refastenable incontinence or enuresis underwear.

Within the context of this specification, each term or phrase below will include the following meaning or meanings.

The term "body side" should not be interpreted to mean in contact with the body of the user, but rather simply means the side that would face toward the body of the user when the garment is applied to the user, regardless of whether the absorbent garment is actually being worn by the user and regardless of whether there are or may be intervening layers between the component and the body of the user. Likewise, the term "garment side" should not be interpreted to mean in contact with the garments of the user, but rather simply means the side that faces away from the body of the user when the garment is applied to the user, and therefore toward any outer garments that may be worn by the user, regardless of whether the absorbent garment is actually being worn by a user, regardless of whether any such outer garments are actually worn and regardless of whether there may be intervening layers between the component and any outer garment.

The term "machine direction" means the direction of flow as the various members and webs progress along the fabrication line and process. It should be understood that various separate members or webs can each be traveling in a machine direction, but with the various machine directions not necessarily being parallel or oriented in the same direction. For example, one web may be traveling along a first machine direction, which is substantially perpendicular to the travel of another web in a second machine direction.

The term "cross direction" means the direction substantially perpendicular to the machine direction.

The term "downstream" means that one item is positioned more closely to the output or finished product end of the machine and/or process relative to another item. Conversely, the term "upstream" means that an item is positioned more closely to the input end of the machine or process relative to another item. For example, the output end is downstream of the input end, and vice versa, the input end is upstream of the output end.

The term "disposable absorbent undergarment" as used herein is an article that is intended to be worn by persons, including infants, toddlers or adults, which is designed for a single or temporary use and is meant to be disposed of after being used once instead of being laundered or dry cleaned for re-use.

The term "attached" refers to the joining, adhering, bonding, connecting, or the like, of two elements. Two elements will be considered to be attached together when they are attached directly to one another or indirectly to one another, such as when each is directly attached to intermediate elements.

The term "disposable" refers to articles which are designed to be discarded after a limited use rather than being laundered or otherwise restored for reuse.

The term "elastomeric" refers to a material or composite which can be elongated by at least 50% of its relaxed length and which will recover, upon release of the applied force, at least 20% of its elongation. It is generally preferred that the elastomeric material or composite be capable of being elongated by at least 100%, more preferably by at least 200%, of its relaxed length and recover, upon release of an applied force, at least 50% of its elongation.

The term "weakening" means to cause to lose strength, such that the area that is weakened is not as strong as the adjacent areas. For example, and without limitation, an area that is weakened may have a lesser tear or tensile strength as compared with the adjacent areas of the web, such that the web is more likely to be torn or broken along the area of weakness rather than the adjacent areas. In this way, the manufacturer can control the area of the web that will be broken, whether such breakage is performed by the end user or at a later time during the manufacturing or fabrication process.

The term "line of weakness" refers to any region or area of weakened material, preferably having a length and which may or may not have a defined width, and can include linear and non-linear patterns, such as curvilinear patterns of weakness, or other shapes, such as circles, rectangles, etc. The line of weakness can include a perforation or other series of cuts, a thinning, or breakage or separation of material, or a strip of a different kind of material bridging between adjacent portions of material, that is more easily torn or broken than the adjacent portions, and which allow the user or manufacturer to separate the adjacent portions along the line of weakness.

The term "altering" refers to changing the modulus of the material. For example, and without limitation, an area that is altered may have a more brittle area as compared with the adjacent areas of the web, such that the web is more likely to be torn or broken along the area of brittleness rather than the adjacent areas. In this way, the manufacturer can control the area of the web that will be broken, whether such breakage is performed by the end user or at a later time during the manufacturing or fabrication process.

The term "altered area" refers to any region or area of altered material, preferably having a length and which may or may not have a defined width, and can include linear and non-linear patterns, such as curvilinear patterns of altered areas, or other shapes, such as circles, rectangles, etc. The altered area can include a pressure bonded or other series of bonds, more brittle areas or a strip of a different kind of material bridging between adjacent portions of material, that is more easily torn or broken than the adjacent portions, and which allow the user or manufacturer to separate the adjacent portions along the altered area.

The terms "longitudinal" and "transverse" have their customary meaning, as indicated by the longitudinal and transverse axes depicted in the Figures. The longitudinal axis lies in the plane of the article and is generally parallel to a vertical plane that bisects a standing wearer into left and right body halves when the article is worn. The transverse axis lies in the plane of the article generally perpendicular to the longitudinal axis.

These terms may be defined with additional language in the remaining portions of the specification.

A method and apparatus for producing a pre-fastened adjustable pant-like disposable absorbent undergarment including a fully or partially severed front body panel is disclosed. The method includes the steps of providing a moving web and passes through a fastener attachment station to define a leading portion of the moving web and a trailing portion of the moving web at a location under the fastener assembly. Finally, the moving web is then passed through a laser cutting station to cut the web, such that the leading portion and trailing portion remains bridged together by the fastener assembly.

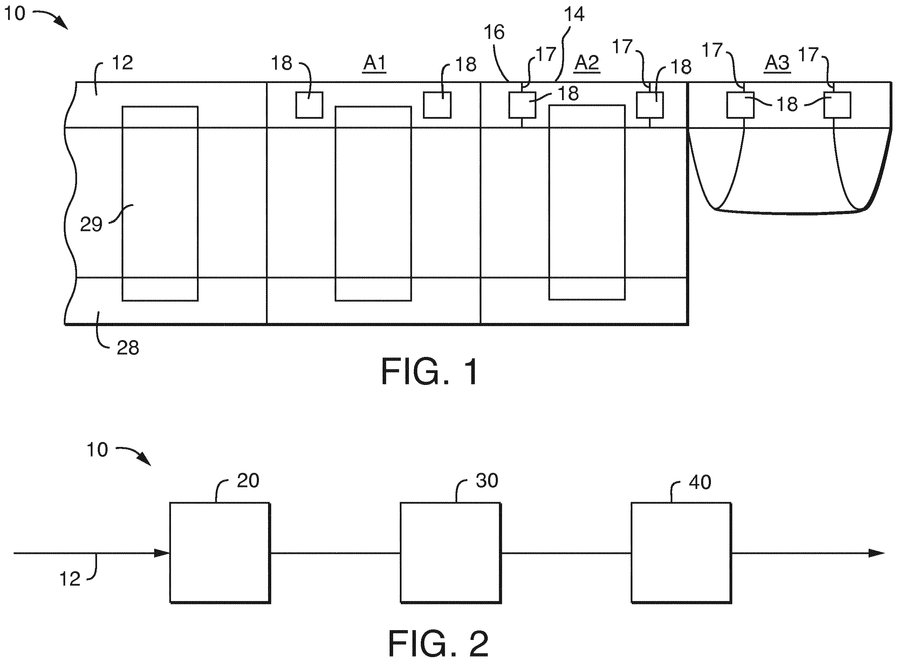

Referring to Figures 1 and 2, a method and apparatus for producing a pre-fastened adjustable pant-like disposable absorbent undergarment is illustrated. A moving web 12 is shown as moving in a machine direction along a process line. The moving web 12 may be used as a front body panel, a back body panel, or both in the disposable absorbent undergarment.

The moving web 12 can be formed from a single piece of material or can be formed as a laminate consisting of two or more layers. The layers of the laminate can be of the same material or different material. In one embodiment, a laminate is formed from a first layer and a second layer. Sandwiched between the first and second layers are two or more elastic strands. Desirably, from two to about a hundred elastic strands can be utilized in the moving web 12 depending upon the overall size of each panel. The elastic strands can be formed from LYCRA, or a similar material. LYCRA is a trademark of INVISTA (Wichita, KS). The diameter and/or cross-sectional configuration of the elastic strands, the decitex (weight in grams per 10,000 meters) of the elastic strands, and the tension imparted into the elastic strands can all be varied to suit one's particular product needs. The exact number of elastic strands that are utilized should be sufficient to ensure that the disposable absorbent undergarment 10 snuggly conforms to the wearer's torso.

The elastic strands can be coated with an adhesive. By adhesively coating each of the elastic strands, instead of slot coating a major portion of the inner surface of at least one of the first and second layers, softer moving webs 12 respectively, can be obtained. Wearers of disposable absorbent undergarments prefer a product that has a softer feel since it is more like underwear.

In other embodiments, the elastomeric nonwoven material comprises an elastomeric film sandwiched between two nonwoven facing layers. U.S. Patent No. 7,803,244 to Siqueira et al., discloses particular examples of elastomeric nonwoven composites suitable for use in the adjustable undergarment described herein.

It should be noted that the moving web 12 can be formed from a breathable or a non-breathable material. Desirably, the moving web 12 is formed from a breathable material or a material that is treated or processed to be breathable. Spunbond and bonded carded webs are two breathable materials that work well as moving web 12 in disposable absorbent undergarments. Bonded carded webs are produced and commercially sold by a variety of vendors. Other materials that can be used to form the moving web 12 include woven and non-woven materials formed from natural or synthetic fibers; polyolefins, such as polypropylene or polyethylene; thermoplastic films; as well as other materials known to those skilled in the art. A metallocene polypropylene works very well since it has a soft feel and can be easily ultrasonically bonded to itself.

The moving web 12 is first passed through a fastener attachment station 30 at a first location A1. The fastener attachment station 30 attaches a fastener assembly 18 over an area that defines a leading portion 14 of the moving web 12 and a trailing portion 16 of the moving web 12 together with a fastener assembly 18. The fastener assembly 18 will maintain the integrity of the web as it continues through the remainder of the process.

Downstream from the fastener attachment station 30, the moving web 12 is passed through a laser cutting station 40. In an exemplary embodiment, the laser cutting station 40 completely separates the leading portion 14 of the moving web 12 and trailing portion 16 of the moving web 12, such that the leading portion 14 and trailing portion 16 remains bridged together by the fastener assembly 18. Desirably, the fastener assembly 18 remains intact with minimal wear or no damage due to the laser cutting only the nonwoven web.

In another embodiment, the laser cutting station 40 cuts the moving web 12 to form a line of weakness between the leading portion 14 of the moving web 12 and the trailing portion 16 of the moving web 12 at a second location A2. This line of weakness could include a series of intermittent perforations. Alternatively, the line of weakness could include scoring the web, or cutting a depth into the material without breaking the material, to provide a weakened area. In these embodiments, the web is not completely severed, but provides a weakened area that a user of the disposable absorbent undergarment may easily break to readjust the garment for a better fit.

The speed of the laser cut, laser intensity, field of view, focal length, and laser wavelength may be optimized by one skilled in the art depending on the speed of the web and the type of material being cut. The laser cutting station 40 may be setup to specific parameters to partially or completely sever the moving web while leaving the fastening assembly 18 intact and without damaging the fastener assembly 18. This allows the web to be separated with the fastening system 18 already in place significantly reducing the opportunity for process interruptions/web breaks. Cutting one of two webs with a standard flex-knife module would be extremely challenging at high speeds while accounting for knife wear. Laser technology offers the ability to accurately maintain its wavelength and intensity.

In other embodiments, the fastener assembly 18 is weakened by the laser as the web is cut with the laser. This may be done by controlling the laser cut, laser intensity, field of view, focal length, and laser wavelength to cut the web, but only scoring the fastener to provide a weakened area. In other words, the laser may cut a depth into the fastener assembly 18 without breaking the fastener assembly 18. A weaker fastener assembly 18 provides for a more flexible fastener assembly 18 that could be easier for a consumer to manipulate.

Panel stability is also an important aspect to maintaining focal distance of the laser. Panel stability may be provided by wrapping the material on a drum, a vacuum conveyor of non-laser cuttable material, or using short span rollers.

Optionally, upstream from the fastener attachment station 30, the moving web 12 is first passed through a web altering station 20. The web altering station 20 forms an altered area in the moving web 12 to define a leading portion 14 of the web 12 and a trailing portion 16 of the moving web 12 connected at the altered area, as shown for example in FIG 2. In a desirable embodiment, the moving web 12 altering station 20 forms a cross-direction altered area, which is preferably linear, in the moving web 12. The altered area can extend across the entire cross-direction width of the moving web 12, or along only a portion thereof. In some embodiments, the altered area can be tapered relative to the longitudinal axis 111, if desired. In addition, the altered area can also be curved. Forming the altered area in the moving web 12 makes the moving web 12 easier to cut with the laser.

In one desirable embodiment, the web altering station 20 is configured as a pressure bonding or flex knife module to pressure bond the moving web 12 through cold flow while changing the modulus, increasing brittleness and maintaining the tensile strength of the moving web 12. In other alternative embodiments, the web altering station 20 can comprise a device for applying heat, thermal energy or ultrasonic energy to the moving web 12 so as to bond the moving web 12 at specific locations, or altered areas. In other preferred embodiments, the web altering station 20 can include a chemical applicator that applies various chemicals, including for example water, to the movingweb 12 to change the modulus at specific locations. Of course, it should be understood that the web altering station 20 can also be configured from combinations of one or more of the above-referenced devices.

Various methods and apparatus for manufacturing absorbent garments and for applying fastener members thereto are disclosed in U.S. Patent No. 6,730,188 entitled "Method and Apparatus For Assembling Refastenable Absorbent Garments," U.S. Patent No. 6,743,321 entitled "Method and Apparatus For Assembling Refastenable Absorbent Garments," U.S. Patent No. 6,686,626 entitled "Method and Apparatus For Assembling Refastenable Absorbent Garments," U.S. Patent No. 6,682,626 entitled "Method and Apparatus For Assembling Refastenable Absorbent Garments," U.S. Patent No. 6,712,922 entitled "Multiple Component Web," U.S. Patent No. 6,730,188 entitled "Method of Assembling Personal Care Absorbent Article," U.S. Patent No. 6,783,487, entitled "Pant-Type Personal Care Articles, and Methods of Making and Using Such Personal Care Articles," U.S. Patent 6,454,888 entitled "Methods of Changing Size of Pant-Type Personal Care Articles Outputted from a Manufacturing Process".

In one embodiment, illustrated in FIG. 1, the moving web 12 is used as a front body panel 112 and bonded to a rear body panel web 28 at side seams 118, 120, wherein the rear body panel web 28 is positioned over the moving web 12 by folding a crotch portion 29 joining or bridging between the moving web 12 and back panel web 28 at a third location A3.

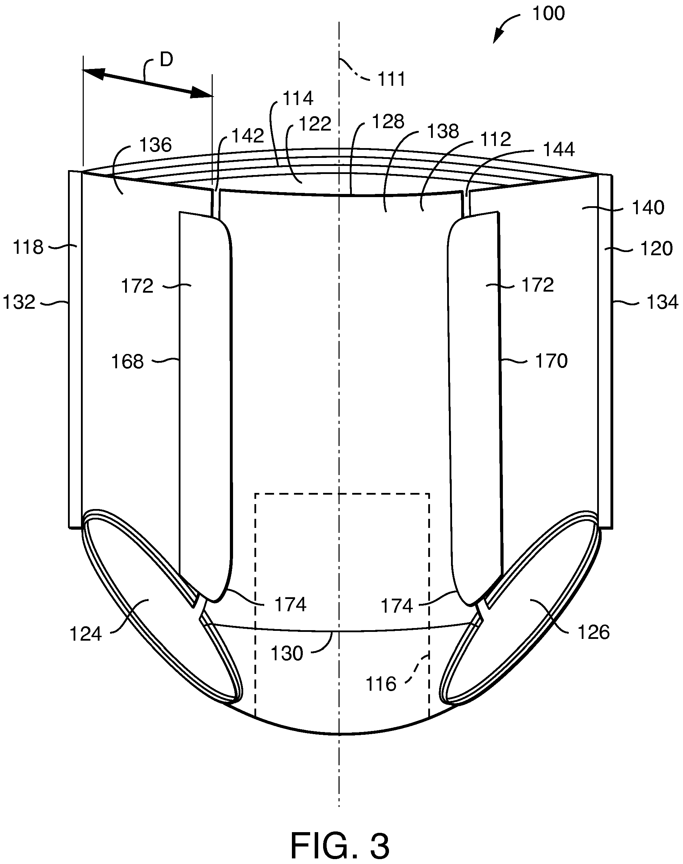

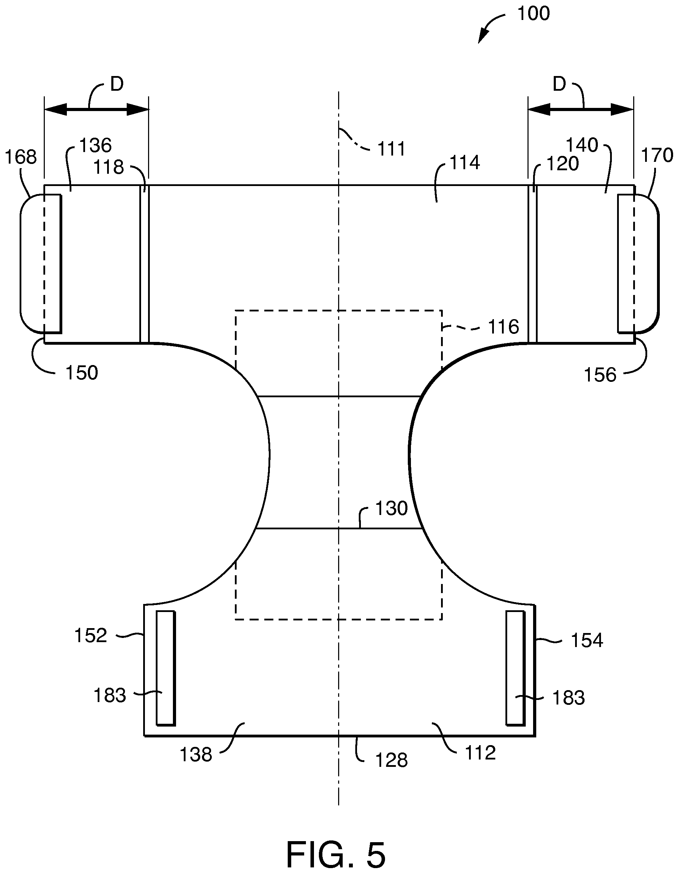

Referring to Figures 3-5, an adjustable pant-like disposable absorbent undergarment 100 having a longitudinal axis 111 made using the method described herein is shown. The adjustable pant-like disposable absorbent undergarment 100 is designed to absorb liquid, semi-solid and/or solid waste discharged from a human being. The adjustable pant-like disposable absorbent undergarment 100 is designed to absorb and/or retain one or more bodily discharges of waste material such as urine, perspiration, excrement, feces, menses, menstrual fluid, as well as other liquid and/or solid waste.

The adjustable pant-like disposable absorbent undergarment 100 includes a front body panel 112, a back body panel 114 and an absorbent assembly 116 secured to the front and back body panels, 112 and 114 respectively. The front and back body panels, 112 and 114 respectively, are joined together by a pair of seams 118 and 120 to form a waist opening 122 and a pair of leg openings 124 and 126.

The front body panel 112 includes a waist edge 128, a crotch edge 130 and a pair of side edges 132 and 134. In a three piece construction wherein an absorbent assembly 116 is secured between the front body panel 112 and the back body panel 114, the crotch edge 130 is well defined. In absorbent undergarments of a different construction, the crotch edge 130 can be an imaginary line transversely drawn between the pair of leg openings 124 and 126 at a location where one considers the front body panel 112 to end. The exact size and configuration of the front body panel 112 can vary to suit a wearer's particular needs. The front body panel 112 has a first side section 136, a middle region 138 and a second side section 140. The front body panel 112 is completely severed between both the first side section 136 and the middle section 138 and the second side section 140 and the middle section 138 creating a first fully severed region, or gap, 142 and a second fully severed region 144. The first side section 136 is aligned adjacent to the side edge 132 at its leading edge forming the seam 120 and the second side section 140 is aligned adjacent to the side edge 134 at its leading edge forming the seam 118. The middle section 138 has a first edge 154 and a second edge 154 and is located between the first and second side sections, 136 and 140 respectively. The terminal edge 150 of the first side section 136 is aligned adjacent to the first edge 152 of the middle section 138 and the terminal edge 150 of the second side section 140 is aligned adjacent to the second edge 154 of the middle section 138. As depicted, the middle section 138 is centrally located and is bifurcated by the longitudinal axis 111 at a midpoint of the front body panel 112.

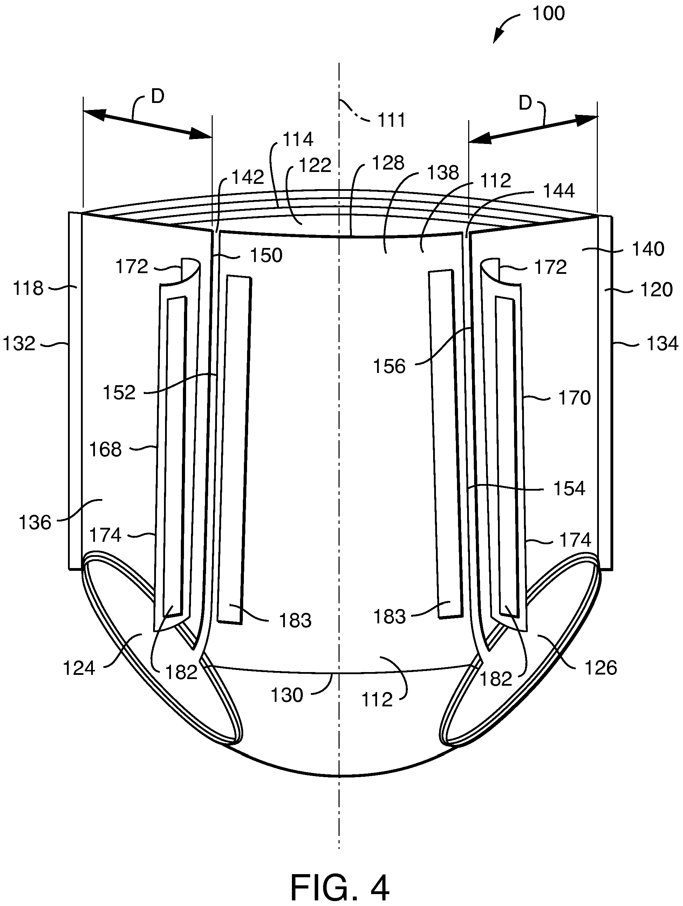

The first and second fully severed regions, 142 and 144, can be linear or non-linear in configuration. In Figures 4-5, the first and second fully severed region, 142 and 144, are shown having a linear or straight configuration. The first and second fully severed regions, 142 and 144, extend longitudinally from approximately the waist edge 128 down to approximately one of the leg openings 124 or 126. The first and second fully severed regions, 142 and 144, can be aligned parallel to the longitudinal axis 111 or be angled thereto. For example, the first and second fully severed regions, 142 and 144, can be tapered relative to the longitudinal axis 111, if desired. In addition, the fully severed regions, 142 and 144, can also be curved.

Still referring to Figures 3-5, each of the first and second fully severed regions, 142 and 144, extend from approximately the waist edge 128 of the front body panel 112 to one of the pair of leg openings 124 and 126. Another way of describing this is to say that the first and second fully severed regions, 142 and 144, extend from approximately the waist edge 128 of the front body panel 112 to approximately the crotch edge 130 of the front body panel 112.

Many adjustable, pant-like disposable absorbent undergarments include a pair of lines of weakness to break to allow for the individual to adjust the size of the pants. This type of adjustable disposable undergarment including the lines of weakness requires an amount of force needed to break the lines of weakness. The pre-fastened adjustable, pant-like disposable absorbent undergarments described herein have the first and second fully severed regions, 142 and 144, that require no force to break allowing for easier use by an elderly individual, caretaker or parent caring for a child.

Still referring to Figures 4-5, each of the first and second fully severed regions, 142 and 144, is shown being aligned parallel to one of the pair of seams 118 and 120. Such an arrangement provides for an aesthetically pleasing appearance to the front body panel 112.

Desirably, in the pre-fastened condition, the first side section 136 and the middle section 138 of front body panel 112 do not overlap, and the second side section 140 and the middle section 138 of the front body panel 112 do not overlap in the severed regions 142, 144. In one exemplary embodiment, a first gap may be formed in the first severed region 142 between the first side section 136 and the middle section 138 of the front body panel 112 in the pre-fastened condition. In this embodiment, a second gap may be formed in the first severed region 142 between the second side section 140 and the middle section 138 of the front body panel 112 in the pre-fastened condition. In another exemplary embodiment, a terminal or outer edge 150 of the first side section 136 abuts against the first edge 152 of the middle section 138 of the front body panel 112 in the pre-fastened condition. In this embodiment, a terminal or outer edge 150 of the second side section 140 abuts against the second edge 154 of the middle section 138 of the front body panel 112 in the pre-fastened condition. In another embodiment, a gap may be formed between one side section and the middle section and the other side section and middle section abut against each other. If a gap is formed between the side sections 136 and 140, and the middle section 138, the gap will desirably be less than 10 mm.

Referring again to Figures 4-5, the front body panel 112 further includes a pair of fastener assemblies 168 and 170. Each fastener assembly 168 and 170 includes a first portion 172 and a second portion 174. The first portion 172 of the fastener assembly 168 can be permanently secured to the first side section 136 of the front body panel 112 and the first portion 172 of the other fastener assembly 170 can be permanently secured to the second side section 140 of the front body panel 112.

In the embodiment shown in Figures 4-5, the first portions 172 of the fastener assemblies 168 and 170 can be securely attached using an adhesive, heat, pressure, a combination of heat and pressure, an ultrasonic bond, a chemical bond or by other means known to those skilled in the art.

Each of the second portions 174 of the fastener assemblies 168 and 170 are depicted as extending almost the entire length of the fully severed portions 142 and 144. This means at least 50% of the distance between the waist edge 30 and the leg opening 124 are covered by the fastener assembly 168. Desirably, the first fastener assembly 168 and second fastener assembly 170 extend at least 65% of the distance between the waist edge 30 and the leg opening 124. More desirably, the first fastener assembly 168 and second fastener assembly 170 extend at least 75% of the distance between the waist edge 30 and the leg opening 124. Even more desirably, the first fastener assembly 168 and second fastener assembly 170 extend at least 85% of the distance between the waist edge 30 and the leg opening 124. Even more desirably, the first fastener assembly 168 and second fastener assembly 170 completely extend at least 95% of the distance between the waist edge 30 and the leg opening 124. The second portions 174 of the first fastener assembly 168 can bridge across the first fully severed region 142 and the second portion 174 of the other fastener assembly 170 can bridge across the second fully severed region 144. By extending substantially the entire length of and bridging the fully severed portions, 142 and 144, the fastener assemblies 168, 170 cover the fully severed portions and provide a more underwear like appearance. The second portions 174 of the fastener assemblies 168 and 170 can be releasably attached to the middle section 138 of the front body panel 112.

Alternatively, it should be evident to those skilled in the art that the first portion 172 of each of the fastener assemblies 168 and 170 could be permanently attached to the middle section 138. In this embodiment, the second portions 174 of the fastener assemblies 168 and 170 can be releasably attached to the side sections of the front body panel 112. In an alternative embodiment, both the first portion 172 and the second portion 174 may be releasably attached. The fastener assembly 168 may be constructed of a non-extensible or an elastomeric material.

Referring now to Figures 4-5, each of the second portions 174 of the fastener assemblies 168 and 170 has an inner surface 180 that contains a fastener 182. The fastener 182 can be a mechanical fastener 182. In Figures 9 and 10, the mechanical fastener 182 is shown as a plurality of fine hooks, such as VELCRO hooks. VELCRO is a trademark of Velcro USA, Inc. (Manchester, NH). The hooks are designed to easily engage and be removed from a material wherein the material has a loose weave pattern or the fibers forming the material will allow the hooks to be attached to them. The mating material is commonly referred to as the loop member of a hook and loop fastener. The middle section 138 of the front body panel 112 may be formed of such a material. When the hooks engage into the middle section 138, a secure but releasable fastener is formed. The hooks can be easily removed from the loop material by pulling the edge of the fastener outward away from the middle section 138. Alternatively, as illustrated in Figures 9 and 10, a fastening component 183 such as a loop material or loose fibers that may be placed anywhere onto the middle section 138 to facilitate the fastening component. Therefore, hook and loop fasteners are referred to as being releasable and can be fastened and released several times. In alternative embodiments, the mechanical fastener 182 constructed of hooks may be placed on to the middle section 138 of the front body panel 112. In this embodiment, the fastening component 183 constructed of a loop material or loose fibers would be placed on the fastener assemblies 168, 170. Both the mechanical fastener 182 and the fastening component 183 may be integral or separately attached.

Referring again to Figures 3-5, one can see that Figure 3 depicts the pair of fastener assemblies 168 and 170 being securely fastened to the middle section 138 of the front body panel 112. If the wearer of the disposable absorbent undergarment 100 or a caregiver wishes to inspect the undergarment 100, he or she would open the pair of fastener assemblies 168 and 170 to the position shown in Figure 4. Since the first and fully severed portions, 142 and 144 are already broken, the middle section 138 of the disposable absorbent undergarment 100 can be easily moved outward away from the wearer's torso. The wearer can then inspect the absorbent assembly 116 to see if it needs to be changed. If so, the disposable absorbent undergarment 100 can be removed from about the wearer's torso and be replaced by another undergarment. If the absorbent assembly 116 is still capable of accepting additional body fluid, the middle section 138 is moved back against the wearer's torso and the pair of fastener assemblies 168 and 170 is refastened to the middle section 138.

The horizontal distance to each of the first and second fully severed regions, 142 and 144, and corresponding first and second fastener assemblies, 168 and 170, as located relative to the pair of side seams 118 and 120 can also vary. A distance "D" is depicted in Figures 4-5 which represents the distance between each of the pair of seams 118 and 120 and the corresponding first and second fully severed regions, 142 and 144 respectively. One can increase the distance "D" by moving the first and second fully severed regions, 142 and 144 respectively, away from the pair of seams 118 and 120. When one increases the distance "D", one may find that it is easier for the wearer of the disposable absorbent undergarment 100 to visually see and identify the first and second fully severed regions, 142 and 144, when user looks down at the front body panel 112. In some embodiments, the first fastener assembly 168 and the second fastener assembly 170 are located near the midpoint between the side seams, 118 and 120, and the cross-sectional midpoint 111 of the front panel 112. Desirably, the first fastener assembly 168 and second fastener assembly 170 are located between 30% and 70% of the distance between the side seam, 118 and 120, and the cross-sectional midpoint 111 of the front panel 112. More desirably, the first fastener assembly 168 and second fastener assembly 170 are located between 35% and 45% of the distance between the side seams, 118 and 120, and the cross-sectional midpoint 111 of the front body panel 112. A manufacturer is free to vary the distance "D" to best suit the size and shape of a particular disposable absorbent undergarment 100 to make sure that the fastener is on the front of the product rather than the side of the product. If the fully severed regions 142, 144 are not parallel to the side seams 118, 120, the midpoint of the fully severed regions 142, 144 in the longitudinal direction may be used to measure "D".

Placement near the midpoint between the side seam and the cross-sectional midpoint 111 of the front body panel 112 facilitates enhanced donning and removal when experiencing a reduced range of motion as well as locating the fastener in a location which is easier for the consumer to see.

The placement of the fastener assemblies 168 and 170 in the front of the product optimizes the ability of the user to fasten the product, while minimizing potential pop-opens during use due to movement of the legs of the user. The placement of the fasteners can be measured in both an unstretched (out of the bag) state or stretched (as in use).

It should be understood that various other embodiments and modifications to the embodiments of the absorbent article described herein which, after reading the description herein, may suggest themselves to those skilled in the art without departing from the scope of the present claims.

## Claims

1. A method of producing pre-fastened disposable garments, comprising:
a. providing a moving web (12) for use as a body panel;
b. attaching a fastener assembly (18) to the moving web (12);
c. cutting the moving web (12) with a laser:
**characterised in that** said cutting is at a position under the fastener (18) assembly defining a leading portion (14) of the moving web (12) and a trailing portion (16) of the moving web (12), such that the leading portion (14) and trailing portion (16) remains bridged together by the fastener assembly (18).

2. The method of claim 1 wherein cutting the moving web (12) comprises completely separating the leading portion (14) of the moving web (12) and the trailing portion (16) of the moving web (12).

3. The method of claim 1 or 2 wherein cutting the moving web (12) comprises a single cut across the web substantially in the cross-machine direction.

4. The method of claim 1 wherein cutting the moving web (12) comprises creating a line of weakness between the leading portion (14) of the moving web (12) and the trailing portion (16) of the moving web (12).

5. The method of claim 1 or 4 wherein cutting the moving web (12) comprises a series of intermittent perforations.

6. The method of claim 1 or 4 wherein cutting the moving web (12) comprises scoring the web to provide a weakened area.

7. The method of any of the preceding claims wherein the fastener assembly (18) is not damaged when the moving web (12) is cut with the laser.

8. The method of any of the preceding claims wherein the fastener assembly (18) is weakened when the moving web (12) is cut with the laser.

9. An apparatus for producing a pre-fastened adjustable pant-like disposable absorbent undergarment using the method of any of the preceding claims comprising:
a. a conveyor to provide a moving web (12);
b. a fastener assembly attachment station (30); and
c. a laser cutting station (40);
**characterised in that**:
the fastener assembly station (30) is for bridging the connected leading portion (14) of the moving web (12) and trailing portion (16) of the moving web (12) together with a fastener assembly (18) that extends over a line of weakness; and
the laser cutting station is for cutting the moving web to define a leading portion (14) of the moving web (12) and a trailing portion (16) of the moving web (12) such that the leading portion (14) and trailing portion (16) remains bridged together by the fastener assembly (18).

## Patentansprüche

1. Verfahren zur Herstellung von vorgeschlossenen Einwegkleidern, umfassend:
a. Bereitstellen einer sich bewegenden Bahn (12) zur Verwendung als Körperteil;
b. Befestigen einer Befestigungsanordnung (18) an der sich bewegenden Bahn (12);
c. Schneiden der sich bewegenden Bahn (12) mit einem Laser:
**dadurch gekennzeichnet, dass** das Schneiden an einer Position unter der Befestigungsanordnung (18) stattfindet, die einen vorderen Abschnitt (14) der sich bewegenden Bahn (12) und einen hinteren Abschnitt (16) der sich bewegenden Bahn (12) derart definiert, dass der vordere Abschnitt (14) und der hintere Abschnitt (16) durch die Befestigungsanordnung (18) miteinander verbrückt bleiben.

2. Verfahren nach Anspruch 1, wobei das Schneiden der sich bewegenden Bahn (12) das vollständige Trennen des vorderen Abschnitts (14) der sich bewegenden Bahn (12) und des hinteren Abschnitts (16) der sich bewegenden Bahn (12) umfasst.

3. Verfahren nach Anspruch 1 oder 2, bei dem das Schneiden der sich bewegenden Bahn (12) einen einzigen Schnitt über die Bahn im Wesentlichen in der Maschinenquerrichtung aufweist.

4. Verfahren nach Anspruch 1, wobei das Schneiden der sich bewegenden Bahn (12) das Erzeugen einer Schwächungslinie zwischen dem vorderen Abschnitt (14) der sich bewegenden Bahn (12) und dem hinteren Abschnitt (16) der sich bewegenden Bahn (12) umfasst.

5. Verfahren nach Anspruch 1 oder 4, wobei das Schneiden der sich bewegenden Bahn (12) eine Reihe von intermittierenden Perforationen umfasst.

6. Verfahren nach Anspruch 1 oder 4, wobei das Schneiden der sich bewegenden Bahn (12) das Ritzen der Bahn umfasst, um einen geschwächten Bereich zu erzeugen.

7. Verfahren nach einem der vorhergehenden Ansprüche, wobei die Befestigungsanordnung (18) nicht beschädigt wird, wenn die sich bewegende Bahn (12) mit dem Laser geschnitten wird.

8. Verfahren nach einem der vorhergehenden Ansprüche, wobei die Befestigungsanordnung (18) geschwächt wird, wenn die sich bewegende Bahn (12) mit dem Laser geschnitten wird.

9. Vorrichtung zur Herstellung einer vorgeschlossenen, verstellbaren, Höschenähnlichen, wegwerfbaren absorbierenden Unterwäsche unter Verwendung des Verfahrens nach einem der vorhergehenden Ansprüche, umfassend:
a. ein Förderband, um eine sich bewegende Bahn (12) zur Verfügung zu stellen;
b. eine Befestigungsanbringungsstation (30); und
c. eine Laserschneidestation (40);
**dadurch gekennzeichnet, dass**:
die Befestigungsanordnungsstation (30) dem Überbrücken des verbundenen vorderen Abschnitts (14) der sich bewegenden Bahn (12) und des hinteren Abschnitts (16) der sich bewegenden Bahn (12) zusammen mit einer Befestigungsanordnung (18), die sich über eine Schwächungslinie erstreckt, dient; und
die Laserschneidstation zum Schneiden der sich bewegenden Bahn dient, um einen vorderen Abschnitt (14) der sich bewegenden Bahn (12) und einen hinteren Abschnitt (16) der sich bewegenden Bahn (12) so zu definieren, dass der vordere Abschnitt (14) und der hintere Abschnitt (16) durch die Befestigungsanordnung (18) überbrückt bleiben.

## Revendications

1. Procédé de fabrication de vêtements jetables pré-attachés, comprenant :
a. la fourniture d'une bande mobile (12) pour l'utilisation comme panneau de corps ;
b. l'attache d'un ensemble de fixation (18) à la bande mobile (12) ;
c. la découpe de la bande mobile (12) avec un laser :
**caractérisé en ce que** ladite découpe se situe dans une position sous l'ensemble de fixation (18) définissant une partie de tête (14) de la bande mobile (12) et une partie de queue (16) de la bande mobile (12), de sorte que la partie de tête (14) et la partie de queue (16) restent pontés ensemble à l'aide de l'ensemble de fixation (18).

2. Procédé selon la revendication 1, dans lequel la découpe de la bande mobile (12) comprend la séparation complète de la partie de tête (14) de la bande mobile (12) et de la partie de queue (16) de la bande mobile (12).

3. Procédé selon la revendication 1 ou 2, dans lequel la découpe de la bande mobile (12) comprend une découpe unique à travers la bande sensiblement dans le sens transversal au sens machine.

4. Procédé selon la revendication 1, dans lequel la découpe de la bande mobile (12) comprend la création d'une ligne fragilisée entre la partie de tête (14) de la bande mobile (12) et la partie de queue (16) de la bande mobile (12).

5. Procédé selon la revendication 1 ou 4, dans lequel la découpe de la bande mobile (12) comprend une série de perforations intermittentes.

6. Procédé selon la revendication 1 ou 4, dans lequel la découpe de la bande mobile (12) comprend la réalisation d'entailles dans la bande pour ménager une zone fragilisée.

7. Procédé selon l'une quelconque des revendications précédentes, dans lequel l'ensemble de fixation (18) n'est pas endommagé lorsque la bande mobile (12) est découpée avec le laser.

8. Procédé selon l'une quelconque des revendications précédentes, dans lequel l'ensemble de fixation (18) est fragilisé lorsque la bande mobile (12) est découpée avec le laser.

9. Appareil pour fabriquer un sous-vêtement absorbant jetable réglable de type culotte pré-attaché selon le procédé selon l'une quelconque des revendications précédentes, comprenant :
a. un convoyeur pour fournir une bande mobile (12) ;
b. un poste d'attache d'ensemble de fixation (30) ; et
c. un poste de découpe au laser (40) ;
**caractérisé en ce que** :
le poste d'ensemble de fixation (30) est destiné à ponter ensemble la partie de tête (14) reliée de la bande mobile (12) et la partie de queue (16) de la bande mobile (12) avec un ensemble de fixation (18) qui s'étend sur une ligne fragilisée ; et
la station de découpe au laser consiste à découper la bande mobile pour définir une partie de tête (14) de la bande mobile (12) et une partie de queue (16) de la bande mobile (12) de sorte que la partie de tête (14) et la partie de queue (16) restent pontées ensemble par l'ensemble de fixation (18).
